# EUROPEAN PATENT APPLICATION

(11) **EP 2 431 394 A1**
(43) Date of publication of application: **21.03.2012**
(21) Application number: 10177468.5
(22) Date of filing: 17.09.2010
(51) Int. Cl.: C08B 15/02, C08B 16/00, C07C 31/26

(54) **Simultaneous hydrolysis and hydrogenation of cellulose**

(71) Applicant: BIOeCON International Holding N.V., Curaçao (AN)
(72) Inventor: Makkee, Michiel, 2628 CN Delft (NL); Moulijn, Jacob Adriaan, 2517 HB Den Haag (NL); Li, Jianrong, 2625 WV Delft (NL)
(74) Representative: Mooij, Maarten

(57) **Abstract**

A process is disclosed for conversion of cellulose in an Ionic Liquid medium. The process is characterized in that a cellulose-containing feedstock and an Ionic Liquid are mixed at a temperature below 80 °C. The mixture is heated to a conversion temperature in the range of 80 °C to 220 °C under a hydrogen atmosphere. The presence of hydrogen during the heating step significantly reduces the formation of degradation products.

## Description

### BACKGROUND OF THE INVENTION

### 1. Field of the Invention

The invention relates generally to a process for converting cellulose in an Ionic Liquid medium, and more particularly to a process wherein cellulose is hydrolyzed to glucose, and glucose is subjected to hydrogenation.

### 2. Description of the Related Art

WO 2009/112,588 discloses a process in which cellulose is dissolved in an inorganic molten salt hydrate. Dissolved cellulose is hydrolyzed to glucose in the presence of an acid catalyst. In a subsequent step glucose is subjected to hydrogenation. In a preferred embodiment the acid catalyst is removed prior to the hydrogenation step.

It has now been discovered that the process of WO 2009/112,588 suffers from losses due to the formation of tar and coke.

Thus, there is a particular need for an improved process for the conversion of cellulose to platform chemicals

### BRIEF SUMMARY OF THE INVENTION

The present invention addresses these problems by providing a process for converting cellulose, said process comprising the steps of:
a. contacting a cellulose-containing feedstock with an Ionic Liquid in a reactor at a temperature below about 80 °C, preferably below 70 °C;
b. admitting a hydrogen-containing gas to the reactor;
c. increasing the temperature of the reactor to a range of 80 °C to 220 °C.

### DETAILED DESCRIPTION OF THE INVENTION

For the purpose of the present invention the term "Ionic Liquid" is defined as an ionic material that is liquid at a temperature below 80 °C, preferably below 70 °C. Preferred are ionic materials that are liquid at room temperature. Ionic Liquids may be organic or inorganic. Many organic Ionic Liquids are subject to hydrogenation reactions under the conditions of steps b. and c. of the process, and are therefore not preferred.

As discussed in more detail below, the cellulose-containing feedstock can be a low cost biomass material. These materials can contain significant amounts of water. Many organic Ionic Liquids must be substantially water free for them to exhibit their cellulose dissolving properties. Use of such Ionic Liquids requires feedstocks that have very low water content. However, drying biomass materials to the point that they can be used with water-intolerant organic Ionic Liquids undesirably adds to the cost of the feedstock.

Preferred Ionic Liquids for use in the process of the present invention are inorganic molten salt hydrates. These materials are chemically stable under the conditions of steps b. and c. of the process, and are tolerant to (in fact, require) the presence of moisture.

Particularly preferred are inorganic molten salts comprising a Zn, Ca or Li cation, and a halogen anion. Most preferred for use in the present process are ZnCl₂ hydrates, in particular ZnCl₂.4H₂O. Mixtures of Ionic Liquids may also be used.

Any cellulose-containing material can be used as the cellulose-containing feedstock in the process of the present invention. Examples include substantially pure cellulose, such as microcrystalline cellulose, or cotton linters, or pre-consumer or post-consumer cotton recycle materials. These feedstocks are particularly suitable for processes employing organic Ionic Liquids.

Other examples of cellulose-containing feedstocks include pre-consumer and post-consumer recycled paper. Paper is generally substantially water free, and therefore suitable as the cellulose-containing feedstock for embodiments of the process employing organic Ionic Liquids. It will be appreciated that paper generally comprises materials other than cellulose. Depending on the grade of the paper it may contain significant amounts of lignin. Many organic Ionic Liquids act as a solvent for lignin. Lignin dissolved in the Ionic Liquid consumes part of the available hydrogen in steps b. and c. of the process, and results in a variety of reaction products. It may be preferred to select an Ionic Liquid that does not dissolve lignin, so that lignin as may be present in the cellulose-containing feedstock can be removed by straightforward separation techniques, such as filtration. It has been found that inorganic molten salt hydrates are desirable selective solvents, in that they dissolve cellulose but not lignin.

Many grades of paper, in particular high gloss printing paper, contain significant amounts of starch. It has been found that the starch component of these grades of paper readily dissolves in the Ionic Liquid. As starch, like cellulose, is a polymer of glucose, the presence of starch in the reaction mixture does not lead to extraneous reaction products.

Ink and pigments used in printing are essentially suspensions of particles in volatile liquids. The pigment particles are generally not soluble in the Ionic Liquid, and can readily be removed from the reaction mixture by filtration.

A highly useful class of cellulose-containing feedstocks is composed of cellulose-containing biomass material. Of particular interest are biomass materials that do not have value for human consumption or as an animal feed. In particular lignocellulosic materials are abundantly available, at low cost. Examples include energy crops, such as sugar cane and switch grass; agricultural waste, such as wheat straw, rice straw, sugar cane bagasse; forestry waste, such as tree branches, bark, wood chips and saw dust; and the like.

These materials primarily comprise cellulose, hemicellulose, lignin, water, and ash. The cellulose, hemicellulose and lignin components are present as a composite material, primarily in the cell walls of the biomass material. Of the three components hemicellulose is removed most easily. Lignin and cellulose, however, are strongly entangled. As one would expect, Ionic Liquids that are capable of dissolving both cellulose and lignin are also capable of dissolving the lignocellulosic composite material.

Surprisingly, Ionic Liquids that dissolve cellulose but that do not dissolve lignin are capable of dissolving the cellulose component of a lignocellulosic composite material. As such, these solvents offer a convenient way to separate lignin from cellulose. Inorganic molten salt hydrates belong to this category of Ionic Liquids.

The organic Ionic Liquids rapidly lose their dissolving power when contaminated with water, and therefore need to be used in a form that is substantially water free. The literature tends to define "substantially water free" as "containing less than 5 wt% water". As a practical matter most organic Ionic Liquids lose their dissolving power when the water content is 1 wt% or higher. Organic Ionic Liquids are not preferred as solvents for biomass materials, which may contain anywhere from 5 wt% to 50 wt% water.

The term "ash" is used for inorganic materials present in biomass materials. The amount of ash varies dramatically, according to the biological species from which the biomass is obtained, the part of the plant, and, to a lesser extent, from the composition of the soil in which the plant was grown. Main components of ash include silicon (in the form of silica); phosphorous (in the form of phosphates); alkali metals (Na, K), in the form of soluble salts; and alkaline earth metals (Ca, Mg), primarily in the form of soluble salts.

The presence of ash in the feedstock presents a problem in that the ash components build up in the Ionic Liquid. Regeneration of the Ionic Liquid generally requires removal of minerals (in addition to the removal of water). An important portion of the ash component of a lignocellulosic biomass feedstock is strongly associated with the lignin component. Removal of undissolved lignin generally results in removal of ash components associated therewith. Accordingly, the use of Ionic Liquids that selectively dissolve the cellulose component of a lignocellulosic composite present the additional advantage of permitting easy removal of a significant portion of the ash component.

Minerals not associated with lignin can be removed by washing the biomass feedstock with water, squeezing free water from the biomass material, or a combination of such measures. Certain biomass materials have low ash content and can be used in the process without requiring a mineral removal step.

Hemicellulose contains many different sugar monomers. For instance, besides glucose, sugar monomers in hemicellulose can include xylose, mannose, galactose, rhamnose, and arabinose. Hemicelluloses contain most of the D-pentose sugars, and occasionally small amounts of L-sugars as well. Xylose is always the predominant sugar monomer, but mannuronic acid and galacturonic acid also tend to be present. Cellulose, by contrast, is a homopolymer of glucose. Unlike cellulose, hemicellulose (also a polysaccharide) consists of shorter chains - 500-3,000 sugar units as opposed to 7,000 - 15,000 glucose molecules per polymer seen in cellulose. In addition, hemicellulose is a branched polymer, while cellulose is unbranched. Hemicellulose, if present in the reaction mixture, reacts in a manner similar to cellulose. But the complex nature of hemicellulose gives rise to a large number of reaction products. If this complexity is undesirable, hemicellulose can be removed from the cellulose-containing feedstock, using techniques well known in the paper industry.

The cellulose-containing feedstock is contacted with the Ionic Liquid at a relatively low temperature, i.e., below 80 °C, preferably below 70 °C. Depending on the temperature and the Ionic Liquid selected, the cellulose component of the feedstock may or may not dissolve in the Ionic Liquid, or it may dissolve partially. Whether or not (partial) dissolution of the cellulose component takes place at this time is not of particular relevance, as any undissolved cellulose will become dissolved during steps b. and c.

An important aspect of the process of the invention is the fact that hydrogen is admitted to the reactor before the temperature of the reactor is raised above 80 °C. It has been found that this measure significantly reduces the formation of tar and char, and significantly increases the yield of desirable reaction products, such as sorbitol. Without wishing to be bound by theory, we believe that glucose, which is formed by hydrolysis of cellulose, is instable in the Ionic Liquid medium. Glucose hydrolysis takes place when the temperature of the reaction mixture is above 80 °C. As hydrogen is present, glucose is hydrogenated to more stable reaction products as it is formed. The formation of tar and char, which are believed to be decomposition products of glucose, is thereby greatly reduced.

It is desirable to operate step c. under increased hydrogen pressure, as doing so increases the hydrogenation rate of glucose. The hydrogen pressure can be in the range of from 1 to 80 bar, preferably in the range of from 10 to 50 bar.

The reaction temperature of step c. can be maintained for a time of from 0.5 hours to 6 hours, preferably from 1.5 to 4 hours. The reaction temperature can be in the range of from 80 °C to 220 °C, preferably from 110 °C to 180 °C.

A catalyst can be present in the reactor during step c. The catalyst can be a hydrogenation catalyst, for example a catalyst containing a metal selected from Pd, Pt, Ni, Co, Ru, Re, Rh, Ir and Fe, and a support, which is preferably carbon, or alternatively zirconia, titania, niobia, silica or tin. The preferred catalyst comprises Ru, for example on a carbon support.

The process of the invention can be used for converting cellulose to a large spectrum of platform chemicals. Examples of such processes are disclosed in WO 2009/112,588, the disclosures of which are incorporated herein by reference. In a preferred embodiment cellulose is converted to sorbitol. Sorbitol can be dehydrated in the Ionic Liquid medium to isosorbide. The conversion to sorbitol can be carried out to a yield of 90% or higher, based on the amount of cellulose in the feedstock. Isosorbide can be removed from the reaction medium using known techniques, such as solvent extraction.

In a preferred embodiment the process comprises the additional step of regenerating the Ionic Liquid medium after the desired reaction product, such as isosorbide, has been removed. This additional regeneration step can comprise removing water from the Ionic Liquid medium. The regeneration step can comprise removing sludge from the Ionic Liquid medium. The term "sludge" as used herein refers to solid reaction products that are insoluble in the Ionic Liquid medium. The term encompasses such reaction products as coke and certain types of char. In general the process can be operated such that little or no coke and char are formed. However, it may be desirable to produce gaseous hydrocarbons under conditions that promote cracking. Such reaction conditions can promote the formation of coke and/or char. The operator of the process may well accept a certain amount of coke yield as a price to pay for a high gas yield, as coke is easily removed from the Ionic Liquid medium. In general, coke removal can be accomplished by passing the Ionic Liquid through a suitable filter medium, such as a bed of silica or alumina. The filter medium can be regenerated by burning off the coke and any other components of the sludge. Heat generated during this regeneration process can be used in the conversion process, in particular in step c.

The removal of water can generally be accomplished by distillation. Step c. is generally carried out under increased pressure, at temperatures exceeding 100 °C. By releasing the pressure while the temperature of the Ionic Liquid medium is maintained above 100 °C, water is flashed off in a process sometimes referred to as flash-distillation.

After regeneration the Ionic Liquid medium may be recycled to step a. of the process. This feature is particularly useful if the process is conducted in continuous mode. It will be understood, however, that the process can be conducted in batch mode as well.

Lignin is insoluble in certain Ionic Liquid media, and partially soluble in others. Undissolved lignin is optionally removed from the process prior to step b. Dissolved lignin is converted to hydrocarbon compounds during step b. Thus, the process of the invention offers flexibility to the operator of the process. The operator may select an Ionic Liquid medium in which lignin is at least partially soluble. The advantage is that a greater portion of the feedstock is converted to hydrocarbons. The mixture of hydrocarbon compounds is more complex if lignin is present in the Ionic Liquid medium during step b. This is not necessarily a disadvantage. For example, if the hydrocarbon products produced by the process are to be used as a gasoline mixing stock, the presence of lignin conversion products tends to increase the octane rating of the mixture.

In an alternate embodiment the operator of the process can select an Ionic Liquid medium in which lignin is substantially insoluble. As a general rule, lignin is insoluble in inorganic molten salt hydrates. It has surprisingly been found that nevertheless these materials are capable of dissolving the cellulose component of a lignocellulosic composite material. This makes it possible to isolate the cellulose portion of a lignocellulosic material, without requiring a separate process, such as the Kraft process, which involves the use of aggressive and environmentally undesirable chemicals.

Undissolved lignin can be removed from the Ionic Liquid medium prior to step b. In this embodiment essentially no lignin is present during step b. As a result, the hydrocarbon mixture produced in the reaction is relatively simple.

The process is particularly suitable for the use of feedstocks comprising lignocellulose. These feedstocks contain, by definition, lignin as a contaminant. Most sources of lignocellulosic materials further comprise water. As explained hereinabove, water can be removed during the regeneration of the Ionic Liquid medium, to avoid undesirable dilution of the medium.

In a particular embodiment of the process of the invention lignin removed from the Ionic Liquid medium can be used to generate hydrogen for use in step c. of the process.

The approximate empirical formula of lignin is C₄₃H₄₃O_{13.} The overall reaction of steam reforming of lignin to hydrogen and carbon dioxide can be represented by the following equation:

2 C₄₃H₄₃O₁₃ + 146 H₂O→ 86 CO₂ + 189 H₂

Steam reforming of 1 kg lignin has the theoretical potential of producing 246 g hydrogen. The composition of lignocellulose varies per plant species and within plants. As a rule of thumb, lignocellulose comprises about one third by weight cellulose, about one third hemicellulose, and about one third lignin. The cellulose components present in one kg dry lignocellulose require about 8.4 g hydrogen for conversion to sorbitol and xylitol, and about 55 g hydrogen for complete conversion to alkanes. The lignin component can produce up to 82 g hydrogen in a steam reforming process. It is clear that the process of the invention can produce a significant excess of hydrogen. The excess can be used as a fuel to provide heat for the process, or can be sold as a valuable commodity.

Many sources of lignocellulosic material further contain inorganic materials. To the extent these materials are insoluble in the Ionic Liquid medium they can readily be removed from the process prior to step b. Inorganic materials that are dissolved in the Ionic Liquid medium can be removed in the regeneration step, for example using solvent extraction.

### EXAMPLE 1

In this comparative example cellobiose (a dimer of glucose) was dissolved in an Ionic Liquid (ZnCl₂.4H₂O) and heated to 130 °C in a nitrogen atmosphere.

Experimental details are as follows:

A 16 mL mini-multi-batch reactor made of Hastelloy was provided with a magnetic stirrer and a K-type thermocouple. The reactor could be placed in an external heater, which could be pre-set to any desired temperature up to 300 °C. The reactor was connected to two gas supplies, N₂ and H₂. Maximum operating pressure of the reactor was 100 bar.

0.5 g D-(+)-Cellobiose (from BioChemika, for microbiology, purity as determined by HPLC >= 99.0%) were dissolved in 6 g ZnCl₂.4H₂O (ACS reagent, purity >= 98.0%, Sigma-Aldrich) at room temperature. The solution was charged to the reactor. The reactor was brought to 30 bar nitrogen pressure, and placed in the external heater which was pre-set at 130 °C. The reactor contents were stirred continuously. After about 15 minutes the reactor temperature stabilized at 5-10 °C below the pre-set heater temperature. This point was taken as t=0.

At the end of the predetermined reaction time, the stirrer was stopped and the reactor was removed from the heater and immersed in a cold water bath. When the reactor temperature reached room temperature the pressure was released. The reaction mixture was removed from the reactor and prepared (diluted and filtrated) for HPLC analysis. The results are reported in Table 1.

**Table 1**

| Reaction Time (min.) | Conversion (%) | Glucose Yield (%) | Fructose yield (%) | Others** (%) | S_{gluccose} (%) |
|---|---|---|---|---|---|
| 10 | 56.9 | 47.9 | 4.2 | 4.8 | 84.2 |
| 30 | >60* | 48.0 | 3.6 | 8.5 | <80 |
| 60 | >60*** | 28.5 | 4.0 | 7.9 | <<48 |

| | | | | | |
|---|---|---|---|---|---|
| Notes: * Extensive amount of degradation products formed. ** Tar was observed, but not reported (hidden in ZnCl₂ peak in HPLC) *** Large amounts of tar were formed. | | | | | |

After a short reaction time of 10 minutes, close to 60% conversion was observed, with 84.2% selectivity to glucose. A longer reaction time of 30 minutes did not increase the glucose yield; instead, degradation products were formed. After a reaction time of 60 minutes large amounts of tar could be visually observed. The glucose yield had decreased by more than half.

### EXAMPLE 2

In this example a solution of cellobiose in an Ionic Liquid was heated in a hydrogen atmosphere.

The same experimental set-up was used as in Example 1. 0.5 g cellobiose were dissolved in 6 g ZnCl₂.4H₂O. The solution was charged to the reactor. 0.125 g of a Ru/C catalyst (5 wt% Ru on carbon), Sigma-Aldrich) were added to the reactor. The reactor was brought to 40 bar hydrogen pressure. Stirring was started (1500 rpm), and maintained throughout the reaction. The reactor was placed in the external heater, which was preheated at 130 °C. After about 15 minutes the reactor temperature stabilized at about 5-10 °C below the heater temperature. This was taken as t=0.

At the end of the predetermined reaction time the stirring was stopped. The reactor was removed from the heater and placed in a cold water bath. After the reactor temperature reached room temperature the pressure was released and a sample of the reaction mixture was prepared (diluted and filtrated) for HPLC analysis. The results are reported in Table 2

**Table 2**

| Reaction time (min) | Conversion (%) | Glucose yield (%) | Sorbitol yield (%) | Others (%) | S_{sorbitol} |
|---|---|---|---|---|---|
| 50 | 100 | 5.9 | 88.7 | 5.4 | 88.7 |
| 360 | 100 | 0 | 97.0 | 3.0 | 97.0 |

Conversion of cellobiose was complete after 50 minutes. Already most of the glucose formed was converted to sorbitol. The sorbitol yield further increased after a reaction time of 360 minutes, at which time no glucose was left in the reaction mixture. Comparison with the results of example 1 reveals that heating of cellobiose in the presence of hydrogen eliminates the formation of degradation products.

### EXAMPLE 3

In this comparative example cellulose was contacted with an Ionic Liquid and heated in a nitrogen atmosphere.

The experimental set-up was the same as in examples 1 and 2. 0.25 g microcrystalline cellulose powder (Sigma-Aldrich) and 6 g ZnCl₂.4H₂O were charged to the reactor at room temperature, and thoroughly mixed to avoid sticking of the mixture to the reactor walls. The reactor was brought to a 40 bar nitrogen pressure, and placed in the external heater, which was preheated at 130 °C. After about 15 minutes the reactor temperature stabilized to about 10 °C below the heater temperature (t=0).

After the predetermined reaction time the stirring was stopped. The reactor was removed from the heater and placed in a cold water bath. After the reactor temperature reached room temperature the pressure was released and a sample of the reaction mixture was prepared (diluted and filtrated) for HPLC analysis. The results are reported in Table 3. The conversion could not be measured accurately due to extensive amounts of tar, which were not measurable in the HPLC chromatogram.

**Table 3**

| Reaction time (min) | Conversion (%) | Glucose yield (%) | Others* (%) | S_{glucose} (%) |
|---|---|---|---|---|
| 120 | >3** | 0.2 | 3.3 | <5.7 |
| 360 | >3** | 0.3 | 3.2 | <8.6 |

| | | | | |
|---|---|---|---|---|
| Notes: * Tar and isomers are not included, as both are hidden in the ZnCl₂ peak of the HPLC chromatogram. ** Based on detected compounds. Actual conversion is much higher, due to tar formation. | | | | |

### EXAMPLE 4

In this example cellulose was contacted with Ionic Liquid; the mixture was heated in the presence of hydrogen.

The experimental set-up was the same as in examples 1-3. 0.25 g microcrystalline cellulose powder (Sigma-Aldrich) and 6 g ZnCl₂.4H₂O and 0.125 g Ru/C catalyst (see example 2) were charged to the reactor at room temperature, and thoroughly mixed to avoid sticking of the mixture to the reactor walls. The reactor was brought to a 40 bar hydrogen pressure, and placed in the external heater, which was preheated at 130 °C. After about 15 minutes the reactor temperature stabilized to about 10 °C below the heater temperature (t=0).

After the predetermined reaction time the stirring was stopped. The reactor was removed from the heater and placed in a cold water bath. After the reactor temperature reached room temperature the pressure was released and a sample of the reaction mixture was prepared (diluted and filtrated) for HPLC analysis. The results are reported in Table 4. Degradation reactions products were visible, but to a much lesser extent than in example 3.

**Table 4**

| Reaction Time (min) | Conversion (%) | Glucose yield (%) | Sorbitol yield (%) | Others* (%) | S_{sorbitol (%)} |
|---|---|---|---|---|---|
| 120 | >2** | 0.1 | 0.8 | 1.4 | <34.8 |
| 360 | >58** | 0.4 | 55.0 | 2.3 | <95 |

| | | | | | |
|---|---|---|---|---|---|
| Notes: * Tar and isomers are not included, as both are hidden in the ZnCl₂ peak of the HPLC chromatogram. ** Based on detected compounds. Actual conversion is higher, due to tar formation. | | | | | |

The experiment is repeated at lower conversion temperatures. The contribution of degradation reactions is considerably lower. The sorbitol yield is considerably higher.

## Claims

1. A process for converting cellulose, said process comprising the steps of:
a. contacting a cellulose-containing feedstock with an Ionic Liquid in a reactor at a temperature below about 80 °C, preferably below 70 °C;
b. admitting a hydrogen-containing gas to the reactor;
c. increasing the temperature of the reactor to a range of 80 °C to 220 °C.

2. The process of claim 1 wherein the Ionic Liquid comprises an inorganic molten salt hydrate.

3. The process of claim 2 wherein the inorganic molten salt hydrate comprises zinc chloride.

4. The process of claim 3 wherein the molten salt hydrate comprises ZnCl₂.4H₂O.

5. The process of any one of the preceding claims wherein the cellulose-containing feedstock comprises lignocellulose.

6. The process of claim 5 wherein the cellulose-containing feedstock further comprises hemicellulose.

7. The process of claim 5 or 6 wherein, prior to step a., lignin and/or hemicellulose are at least partially removed from the cellulose-containing feedstock.

8. The process of claim 5 or 6 wherein, prior to step b. undissolved components of the feedstock are removed from the Ionic Liquid.

9. The process of claim 8 wherein the undissolved components comprise lignin, and lignin removed from the Ionic Liquid is used to generate hydrogen.

10. The process of any one of the preceding claims wherein during step c. the hydrogen pressure is in the range of from 1 bar to 80 bar, preferably from 10 bar to 50 bar.

11. The process of any one of the preceding claims wherein, after step c. the temperature of the reactor is maintained in a range of from 80 °C to 220 °C during a time of from 0.5 hours to 6 hours.

12. The process of claim 11 wherein the temperature of the reactor is maintained in a range of from 110 °C to 180 °C.

13. The process of claim 12 or claim 11 wherein the temperature of the reactor is maintained in the range during a time of from 1.5 to 4 hours.

14. The process of any one of the preceding claims wherein a catalyst is present in the reactor.

15. The process of claim 14 wherein the catalyst is a hydrogenation catalyst.

16. The process of claim 15 wherein the catalyst comprises a noble metal.

17. The process of claim 16 wherein the catalyst comprises Ru.

18. The process of claim 17 wherein the catalyst comprises Ru on a carbon support.

19. The process of any one of the preceding claims wherein cellulose is converted to sorbitol.

20. The process of claim 19 wherein cellulose is converted to sorbitol at a yield of more than 90%.

21. The process of claim 20 wherein the sorbitol is removed from the Ionic Liquid, and the Ionic Liquid is regenerated after removal of the isosorbide.
